# DEMANDE DE BREVET EUROPEEN

(11) **EP 4 360 627 A1**
(43) Date de publication de la demande: **01.05.2024**
(21) Numéro de dépôt: 22306622.6
(22) Date de dépôt: 26.10.2022
(51) Int. Cl.: A61K 31/06, A61K 31/4425, A61P 19/02, C07K 16/40, C12N 15/113

(54) **TRAITEMENT DE L'ARTHROSE PAR DES INHIBITEURS DE COMT**

(71) Demandeur: Institut National de la Santé et de la Recherche Médicale (INSERM), 75013 Paris (FR); Université de Caen Normandie, 14000 Caen (FR); Centre Hospitalier Universitaire de Caen Normandie, 14000 Caen (FR); Université Paris Cité, 75006 Paris (FR)
(72) Inventeur: BAUGE, Catherine, 14032 CAEN CEDEX 5 (FR); AURY-LANDAS, Juliette, 14074 CAEN CEDEX 5 (FR); BROCHARD, Sybille, 14032 CAEN CEDEX 5 (FR); BOUMEDIENNE, Karim, 14032 CAEN CEDEX 5 (FR); GROSO, Anthony, 45000 ORLÉANS (FR); BRIET-ROCHOUX, Quitterie, 14000 CAEN (FR); COHEN-SOLAL, Martine, 75010 PARIS (FR); HAY, Éric, 75010 PARIS (FR); BOUCHEMLA, Zohra, 75010 PARIS (FR)
(74) Mandataire: Plasseraud IP

(57) **Abrégé**

L'invention se rapporte à un inhibiteur de de la catéchol O-méthyltransférase (COMT) pour une utilisation dans le traitement de l'arthrose, dans lequel l'inhibiteur est destiné à ralentir de la progression de l'arthrose et à réduire la douleur induite par l'arthrose, ainsi qu'à une composition pharmaceutique comprenant un inhibiteur de COMT pour le traitement de l'arthrose.

## Description

### Domaine technique

La présente invention vise à fournir une nouvelle stratégie pour le traitement de l'arthrose.

### Arrière-plan technologique

L'arthrose ou arthropathie chronique dégénérative est une maladie articulaire chronique caractérisée par une détérioration structurale du cartilage articulaire. Les articulations les plus touchées sont, en dehors du rachis, le genou, la main et la hanche.

Les symptômes de cette pathologie peuvent varier selon l'articulation concernée mais sont en général caractérisés par une douleur persistante associée à une gêne fonctionnelle, c'est-à-dire une limitation de la mobilité de l'articulation concernée.

Le cartilage articulaire est un tissu conjonctif composé d'un seul type de cellules, les chondrocytes, qui sécrètent une matrice extracellulaire dont ils assurent le renouvellement. Cette matrice est essentiellement formée d'eau, de protéoglycanes et de collagène.

Les chondrocytes maintiennent un équilibre entre dégradation et restauration de la matrice extracellulaire. Ces mécanismes sont complexes et notamment régis, pour la dégradation, par des cytokines pro-inflammatoires telles que TNF-a et IL-1 beta, et pour la restauration, par des cytokines modulatrices et des facteurs de croissance, notamment IGF-1, TGF- beta.

La destruction arthrosique du cartilage résulte d'une rupture de cet équilibre entre dégradation et restauration de la matrice extracellulaire. Plusieurs facteurs peuvent favoriser cette rupture de l'homéostasie de la matrice notamment des facteurs mécaniques, liés par exemple a une hyperpression au niveau du cartilage ou des microtraumatismes répétés, ou alors des facteurs métaboliques, génétiques, hormonaux, ou encore le vieillissement. L'initiation du processus arthrosique reste encore à ce jour mal comprise.

A l'heure actuelle, il n'existe pas de traitement efficace et durable pour traiter l'arthrose et son évolution. Les traitements utilisés sont des traitements uniquement symptomatiques à action immédiate ou retardée sur la douleur ressentie, le plus souvent des analgésiques, des anti-inflammatoires et des infiltrations intra-articulaires d'acide hyaluronique ou de corticoïdes.

Le développement de nouveaux antalgiques pour soulager la douleur arthrosique chez les patients intolérants ou réfractaires aux traitements antalgiques standard est complexe. En particulier, des anticorps anti-NGF (abrégé de l'anglais Nerve Growth Factor), qui ciblent une neurotrophine intervenant dans la transduction du signal douloureux, ont fait l'objet de nombreuses études cliniques. Ces anti-NGF ont montré un effet anti-douleur très net, qui est parfois, de manière inattendue et encore inexpliquée, associé à une accélération de la progression de l'arthrose. Il n'existe pas de corrélation directe entre réduction de la douleur et réduction de la progression de la destruction de l'articulation.

Le seul traitement curatif efficace sur la mobilité et les douleurs est le traitement chirurgical par pose de prothèse. La chirurgie est le plus souvent limitée aux articulations de la hanche, du genou ou de l'épaule, très rarement les petites articulations telles les mains ou les pieds. Si la prothèse de hanche est le traitement chirurgical de référence car il donne de très bons résultats, le remplacement prothétique des autres articulations n'est pas satisfaisant en termes de mobilité et de douleurs. De plus, les prothèses utilisées ont une durée de vie limitée entre 15 et 20 ans, et il faut les remplacer si elles sont mises en place chez des patients jeunes.

Actuellement, l'arthrose concernerait environ neuf à dix millions de personnes en France. Compte tenu du vieillissement de la population ainsi que de l'augmentation de la prévalence de l'obésité dans les pays développés, une très forte augmentation du nombre de patients arthrosiques est attendue dans les années à venir. Il devient nécessaire de trouver de nouvelles stratégies thérapeutiques capables de ralentir la destruction du cartilage chez le sujet arthrosique, tout en réduisant les douleurs articulaires.

### Résumé

L'objectif de la présente invention est de fournir de nouveaux composés pouvant être utilisés dans le traitement de l'arthrose.

Les inventeurs ont montré que, de manière surprenante, l'enzyme COMT est exprimée par les chondrocytes des articulations, et que, de manière plus surprenante encore, des inhibiteurs de COMT administrés au niveau de l'articulation sont capables de ralentir ou d'inhiber la progression de la destruction du cartilage, et de réduire la douleur associée à l'arthrose via une action locale et directe au niveau de l'articulation, par l'intermédiaire d'un mécanisme qui n'implique pas le système nerveux central ou périphérique.

Ainsi, selon un premier objet l'invention concerne un inhibiteur de la catéchol O-méthyltransférase (COMT) pour une utilisation dans le traitement de l'arthrose.

Avantageusement, l'inhibiteur est destiné à ralentir de la progression de l'arthrose et à réduire la douleur induite par l'arthrose.

L'inhibiteur peut être choisi parmi un composé dérivé du nitrocatéchol, un anticorps ou un ARN interférent.

Avantageusement, l'inhibiteur est un dérivé du nitrocatéchol comprenant une partie catéchol portant un groupe nitro en position ortho ou para par rapport à l'un des groupes hydroxy de la partie catéchol.

Le dérivé du nitrocatéchol est de préférence choisi dans le groupe constitué par la tolcapone, l'entacapone, l'opicapone, la nitecapone, la nebicapone, et leurs mélanges.

L'inhibiteur est de préférence destiné à être administré par voie intra-articulaire, orale, ou sous-cutanée.

De manière préférée, l'inhibiteur est destiné à être administré par voie intra-articulaire.

L'inhibiteur peut être administré en combinaison avec une ou plusieurs substances actives choisies dans le groupe constitué des antalgiques, des anti-inflammatoires non stéroïdiens, des anti-inflammatoires stéroïdiens, et des anti-arthrosiques, typiquement des anti-arthrosiques symptomatiques d'action lente ou des chondroprotecteurs.

L'invention concerne également une composition pharmaceutique comprenant un ou plusieurs inhibiteurs de COMT tels que définis dans le premier objet de l'invention, et un excipient pharmaceutiquement acceptable, pour une utilisation dans le traitement de l'arthrose.

De manière préférée, la composition pharmaceutique est destinée à être administrée par voie intra-articulaire, orale, ou sous-cutanée.

La composition pharmaceutique peut comprendre en outre une ou plusieurs autres substances actives choisies dans le groupe constitué des antalgiques, des anti-inflammatoires non stéroïdiens, des anti-inflammatoires stéroïdiens et des anti-arthrosiques.

De manière préférée, la composition pharmaceutique est destinée à être administrée par voie intra-articulaire.

La composition pharmaceutique peut comprendre l'inhibiteur et des anti-arthrosiques tels que le plasma riche en plaquettes (PRP), la chondroïtine, et la glucosamine.

La composition pharmaceutique peut être formulée sous la forme d'une composition à libération prolongée.

La composition pharmaceutique peut comprendre l'inhibiteur dispersé dans une fraction polymérique comprenant un polymère biodégradable et/ou biocompatible de préférence choisi dans le groupe constitué par l'acide hyaluronique, le collagène, le chitosane, l'acide polylactique (PLA), l'acide glycolique, les copolymères d'acide lactique et d'acide glycolique (PLGA), les polyoxalates, la polycaprolactone, les polyesters, et leurs mélanges.

### Brève description des dessins

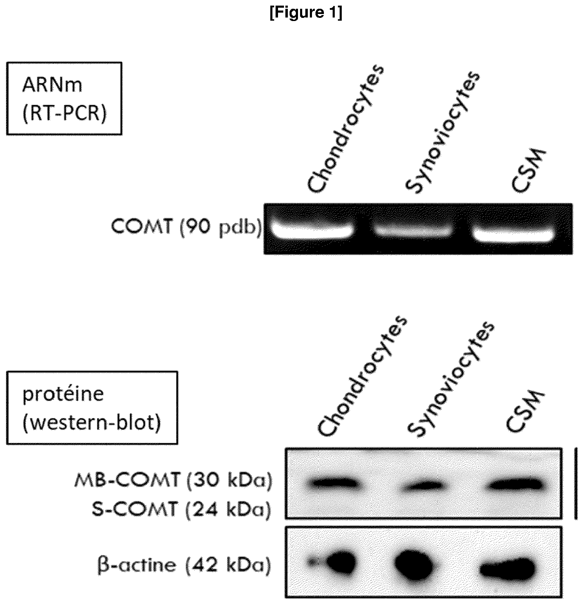

[Figure 1] : Etude de l'ARN messager et des protéines dans des chondrocytes et des synoviocytes articulaires humains. Les résultats montrent que COMT est exprimé (aussi bien ARNm que protéine) dans les cellules présentes au niveau de l'articulation (échantillons humains)

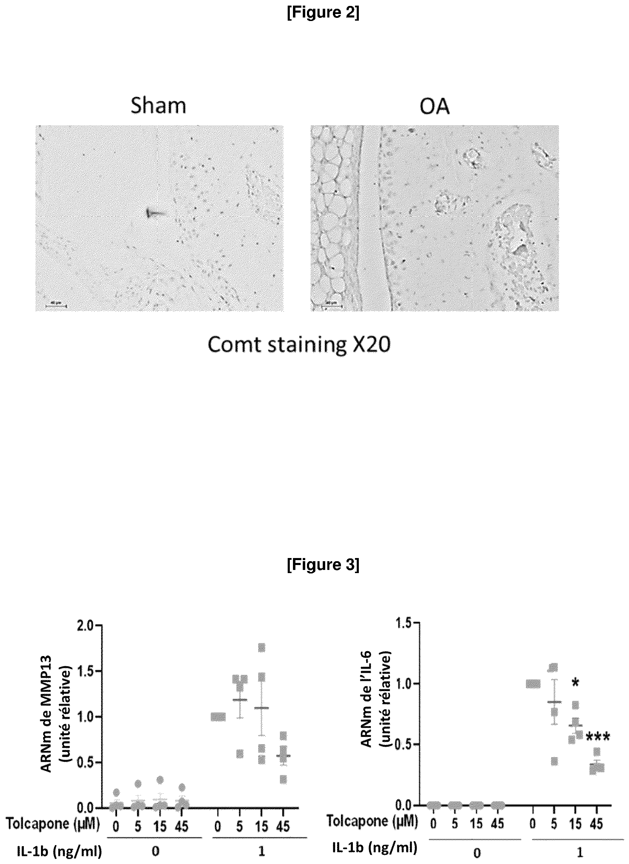

[Figure 2] : Coupes histologiques de souris arthrosiques et de souris normales (modèle DMM). Les résultats montrent que l'expression de *COMT* est augmentée dans l'articulation de souris arthrosiques par rapport à des souris normales (modèle DMM).
[Figure 3] : A. Etude de l'expression d'ARNm codant pour MMP13 dans des chondrocytes murins. B. Etude de l'expression d'ARNm codant pour IL-6 dans des chondrocytes murins. Les résultats montrent que l'inhibition pharmacologique de COMT par le tolcapone réduit l'expression de gènes impliqués dans le processus arthrosique (inflammation, catabolisme) dans des chondrocytes articulaires humains.

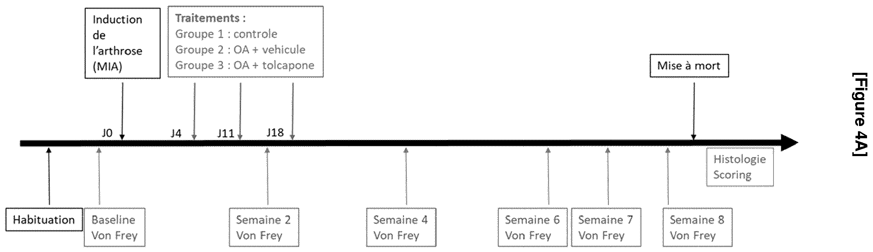

[Figure 4A] : Schéma illustrant le protocole comprenant l'induction de l'arthrose dans un modèle murin d'arthrose (souris MIA), le suivi de la douleur par la technique Von Frey à aux semaines 2, 4, 6, 7 suivant l'induction de l'arthrose, et la mise à mort à la semaine 9, pour l'étude de la dégradation du cartilage par histologie.

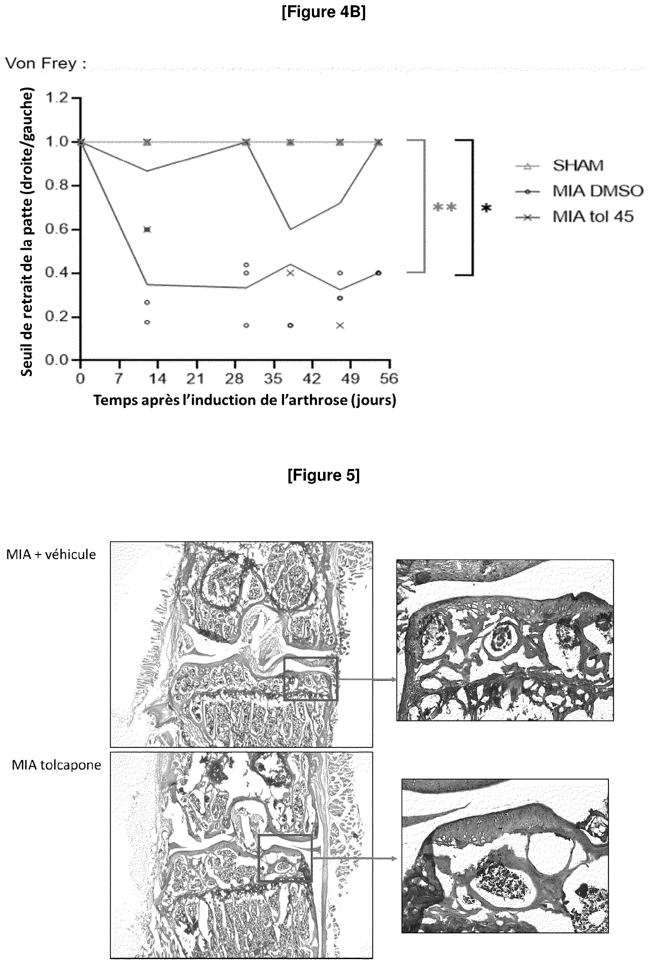

[Figure 4B] : Schéma illustrant la douleur évaluée par la technique de Von Frey en fonction du temps (jour 0= induction de l'arthrose). Les résultats montrent que des injections intra-articulaires d'un inhibiteur pharmacologique de COMT (tolcapone) réduit la douleur dans un modèle murin d'arthrose (MIA).
[Figure 5] : Coupes histologiques de genoux de souris dans un modèle murin d'arthrose (souris MIA) avec et sans injection de topalcone. Les résultats montrent que des injections intra-articulaires d'un inhibiteur pharmacologique de COMT (tolcapone) réduisent la progression de l'arthrose dans un modèle murin d'arthrose (MIA).

### Description détaillée

La catéchol O-méthyltransférase (COMT) est l'enzyme responsable de la O-méthylation des neurotransmetteurs endogènes et des substances xénobiotiques et hormones incorporant des structures catécholiques. Les catécholamines sont une famille de molécules qui ont un rôle d'hormones ou de neurotransmetteurs. Les principales catécholamines présentes chez l'homme sont l'adrénaline (ou épinéphrine), la noradrénaline (ou norépinéphrine) et la dopamine. Elles sont produites par les glandes surrénales et par les neurones post-ganglionnaires du système nerveux parasympathique, par une enzyme appelée tyrosine hydroxylase (TH). Les catécholamines sont dégradées par la COMT qui catalyse le transfert d'un groupement méthyle provenant de la S-adénosyl-L-méthionine (SAM) vers un groupe hydroxyle d'un noyau catéchol. COMT existe sous deux formes, une forme longue membranaire qui permet l'arrêt de la neurotransmission synaptique dopaminergique et noradrénergique et une forme courte cytoplasmique soluble responsable de l'élimination des catéchols exogènes.

La COMT est une cible biologique médicamenteuse pour le traitement de divers troubles du système nerveux central et périphérique, notamment la maladie de Parkinson, la dépression, la schizophrénie et d'autres maladies liées à une carence en dopamine.

En particulier, les médicaments de la classe thérapeutique des inhibiteurs de la catéchol-O-méthyltransférase (I-COMT) sont indiqués dans la prise en charge de la maladie de Parkinson, comme adjuvant pour prolonger la durée d'action de médicaments contenant des précurseurs de dopamine tels que la L-Dopa. Ainsi, les ICOMT utilisés dans la maladie de Parkinson sont systématiquement prescrits en association avec la L-DOPA, et n'ont pas d'effet symptomatique en monothérapie.

Les inventeurs ont montré que les inhibiteurs de COMT pouvaient avoir un effet thérapeutique dans le cadre d'une toute autre classe de pathologies, à savoir l'arthrose.

Un rôle de COMT dans la douleur, en particulier dans la douleur liée à l'arthrose a été mentionné par quelques auteurs (voir par exemple Meurs et al., Arthritis & Rheumatism. 60 628-629. 2009 ; Schutte et al., Research in Gerontological Nursing. Vol. 13, No. 4. 2020; Martire et al., Scandinavian Journal of Pain. 10 6-12. 2016 ; Govil et al., Eur J Pain. 24 398-412. 2020) mais était cependant contesté (voir par exemple Olesen et al., Pain Pract. 18 587-596. 2018 ; Neogi et al., Ann Rheum Dis. 73 315-317. 2014). Toutefois, il était supposé que le mécanisme des inhibiteurs de COMT sur la douleur était lié à un effet sur la perception de la douleur par l'intermédiaire du système nerveux. Autrement dit, les inhibiteurs de COMT étaient suggérés comme de simples analgésiques. Jamais un rôle direct de COMT dans l'articulation n'avait été suggéré. Avant les travaux des inventeurs, l'enzyme COMT n'avait jamais été investiguée comme cible pour le traitement de l'arthrose, et en particulier l'expression de COMT au sein de l'articulation n'avait jamais été investiguée.

Les inventeurs ont à présent démontré que, de manière surprenante, la COMT est exprimée dans des chondrocytes articulaires, et que de manière encore plus surprenante, l'inhibition locale et directe de la COMT au niveau de l'articulation permet de réduire, de ralentir ou d'inhiber la progression de la destruction du cartilage simultanément à une réduction de la douleur.

Les inventeurs ont ainsi montré que des inhibiteurs de COMT sont capables d'inhiber l'expression de gènes impliqués dans le processus de destruction arthrosique dans des chondrocytes articulaires, en particulier le gène codant pour une cytokine pro-inflammatoire telles que l'interleukine-6, et une métalloprotéase telles que MMP-13.

Les inventeurs ont ainsi mis en évidence une action inhibitrice directe des inhibiteurs de COMT sur plusieurs facteurs impliqués dans la destruction du cartilage et l'inflammation lors du processus arthrosique.

De manière importante, les inventeurs ont montré que des inhibiteurs de COMT administrés par voie intra-articulaire sont capables ralentir la progression de la dégradation du cartilage arthrosique et de diminuer la douleur ressentie par le sujet.

Ils ont aussi montré que des inhibiteurs de la COMT administrés au niveau de l'articulation sont également capables de réduire la douleur associée à l'arthrose via une action locale et directe au niveau de l'articulation, par un mécanisme qui n'implique pas le système nerveux central ou périphérique.

L'utilisation de ces inhibiteurs représente donc une nouvelle stratégie de traitement de l'arthrose. Cette stratégie est d'autant plus avantageuse que plusieurs inhibiteurs de COMT ont déjà été approuvés dans d'autres applications thérapeutiques.

### Premier objet de l'invention

Selon un premier objet, la présente invention concerne donc un inhibiteur de COMT pour une utilisation dans le traitement de l'arthrose.

### Inhibiteur de COMT

Dans la présente invention, l'expression « inhibiteur de COMT » ou plus simplement ci-après « inhibiteur » se réfère à tout composé capable d'inhiber l'activité enzymatique COMT ou bien l'expression de l'enzyme COMT au sein de chondrocytes articulaires.

Selon un mode de réalisation, l'inhibiteur de COMT est capable de ralentir ou d'inhiber l'activité enzymatique de la COMT.

De manière préférée, l'inhibiteur utilisé selon l'invention est un dérivé du nitrocatéchol.

Par dérivé du nitrocatéchol, on entend un composé comprenant une partie catéchol portant un groupe nitro en position ortho ou para par rapport à l'un des groupes hydroxy de la partie catéchol.

L'inhibiteur utilisé selon l'invention est de préférence un dérivé du nitrocatéchol choisi dans le groupe constitué par la tolcapone, l'entacapone, l'opicapone, la nitecapone, la nebicapone, et leurs mélanges.

L'inhibiteur utilisé selon l'invention peut également être un anticorps bloquant l'activité de COMT.

Le terme « anticorps » désigne une molécule d'immunoglobuline, c'est-à-dire une molécule qui contient un site de liaison à un antigène capable de se lier de manière immunospécifique à un antigène. Dans la présente invention, le terme "anticorps" englobe non seulement les molécules d'anticorps entières, mais aussi les fragments d'anticorps.

Selon un autre mode de réalisation, l'inhibiteur est capable de ralentir ou d'inhiber l'expression de la COMT. Un tel inhibiteur peut être un ARN interférent complémentaire à la séquence de l'ARN messager de COMT.

La stratégie de l'ARN interférence est bien connue de l'homme du métier. Cette technique fait appel à un processus se déroulant de façon naturelle dans les cellules animales, comprenant notamment la réduction ou l'extinction (« silencing ») de l'expression gène ciblé. La formation d'ARNs double brin conduit à la dégradation spécifique par la machinerie cellulaire de l'hôte de transcrits complémentaires de cet ARN double brin. Ainsi en ciblant COMT par ARN interférence, il est possible de diminuer le niveau d'expression ou de supprimer complètement l'expression du gène COMT dans les cellules ciblées.

L'inhibiteur utilisé selon l'invention est de préférence choisi dans le groupe constitué par la tolcapone, l'entacapone, l'opicapone et leurs mélanges.

### Méthodes de traitement

Le terme « traitement » désigne tout acte permettant de diminuer, de supprimer ou de retarder les symptômes associés à une pathologie chez un sujet à traiter. Il comprend aussi bien un traitement curatif qu'un traitement prophylactique d'une maladie. Un traitement curatif est défini par un traitement aboutissant à une guérison ou un traitement allégeant, améliorant et/ou éliminant, réduisant et/ou stabilisant les symptômes d'une maladie ou la souffrance qu'elle provoque. Un traitement prophylactique comprend aussi bien un traitement aboutissant à la prévention d'une maladie qu'un traitement réduisant et/ou retardant l'incidence d'une maladie ou le risque qu'elle survienne. Dans le cadre de la présente invention, le terme « traitement » se réfère plus particulièrement au ralentissement ou à l'inhibition de la destruction du cartilage dans le cadre du processus arthrosique.

Ainsi, l'inhibiteur utilisé selon l'invention permet le ralentissement ou l'inhibition de la destruction du cartilage dans le cadre du processus arthrosique.

Dans la présente invention, un « chondroprotecteur » ou « antiarthrosique de fond » désigne un médicament permettant de ralentir ou inhiber le processus de destruction dégradation du cartilage.

Avantageusement, l'inhibiteur utilisé selon l'invention permet également la réduction de la douleur causée par l'arthrose, en particulier sans action sur le système nerveux central ou périphérique.

Avantageusement, l'inhibiteur utilisé selon l'invention peut donc être utilisé à la fois à titre d'agent chondroprotecteur et d'agent antidouleur.

Selon un mode de réalisation, l'inhibiteur est utilisé pour le traitement de l'arthrose, dans lequel le traitement comprend le ralentissement de la destruction du cartilage et la réduction de la douleur associée à l'arthrose. Le traitement comprend notamment la réduction de l'expression de gènes impliqués dans le catabolisme tels que MMP13, la réduction de l'expression de gènes impliqués dans l'inflammation tels que l'IL-6, et la stimulation l'expression et la sécrétion de collagène tel que COL2, au niveau de l'articulation.

Comme on le verra ci-après, l'inhibiteur selon l'invention est de préférence formulé de manière à être administré directement au niveau de l'articulation, c'est-à-dire de manière intra-articulaire.

La présente invention concerne également l'utilisation d'un inhibiteur selon l'invention, de préférence un dérivé du nitrocatéchol notamment choisi dans le groupe constitué par la tolcapone, l'entacapone, l'opicapone et leurs mélanges, pour la fabrication d'un médicament destiné au traitement de l'arthrose, dans laquelle le médicament est de préférence administré par voie intra-articulaire.

La présente invention concerne en outre une méthode de traitement de l'arthrose chez un sujet, ladite méthode comprenant l'administration d'une dose thérapeutiquement efficace d'un inhibiteur selon l'invention audit sujet, dans laquelle l'inhibiteur selon l'invention est de préférence un dérivé du nitrocatéchol notamment choisi dans le groupe constitué par la tolcapone, l'entacapone, l'opicapone et leurs mélanges, et dans laquelle l'inhibiteur selon l'invention est de préférence administré audit sujet par voie intra-articulaire.

### Arthrose

Au sens de la présente invention, le terme « arthrose » se réfère à toute pathologie articulaire chronique se traduisant par une détérioration structurale du cartilage articulaire. Le terme « arthrose » inclut ainsi notamment l'arthrose primaire ou primitive, dans laquelle le sujet est atteint d'arthrose sans cause identifiable ni prédisposition particulière, en particulier sans cause traumatique ou maladie inflammatoire associée. Le terme « arthrose » inclut également l'arthrose secondaire, dans laquelle le sujet a subi un traumatisme au niveau d'une articulation (l'arthrose est alors désignée sous le terme « arthrose traumatique ») par exemple lors d'un accident de la route, ou bien est atteint d'une maladie prédisposant à l'arthrose, par exemple les rhumatismes inflammatoires chroniques tels que polyarthrite rhumatoïde, spondyloarthropathie, rhumatisme microcristallin, ou des maladies métaboliques tels que l'obésité, le diabète, ou l'hémochromatose. De manière plus générale, dans la présente invention, le terme « arthrose » comprend également toutes les pathologie articulaires dans lesquelles on observe une détérioration structurale du cartilage articulaire telles que la polyarthrite rhumatoïde, la goutte ou le rhumatisme psoriasique, ainsi que les maladies qui affectent les articulations et génèrent des douleurs telles que l'hémochromatose, les dysplasies articulaires et les maladies rares du squelette.

La suspicion d'arthrose se fait à l'interrogatoire et à l'examen clinique. Le patient décrit des douleurs articulaires mécaniques, de localisation précise. Douleurs le plus souvent reproduites à l'examen clinique.

Le diagnostic d'arthrose repose sur de l'imagerie, en particulier par radiographie standard, scanner, ou IRM.

La radiographie standard permet de retrouver la principale caractéristique de l'arthrose, à savoir la diminution de la quantité du cartilage (le terme radiologique le plus couramment utilisé est celui de pincement articulaire), et selon le stade de l'arthrose, des atteintes osseuses telles que l'ostéosclérose (durcissement de l'os immédiatement sous le cartilage, image blanchie à la radio), la présence d'ostéophytes (excroissances osseuses à la radio), et/ou de géodes (kystes dans l'os sous le cartilage visibles en radio).

Une définition radiologique repose sur la classification de Kellgren et Lawrence (Kellgren JH , Lawrence JS. Radiologcal assessment of osteoarthritis. Ann Rheum Dis, 1957, 16 : 494-502). Le score de Kellgren et Lawrence est un index composite prenant en compte notamment les ostéophytes et le pincement de l'interligne articulaire. Cette classification comporte 4 stades : arthrose douteuse, minime, certaine, évoluée. Le stade 2 est habituellement retenu comme seuil diagnostic. Les stades radiologiques de l'arthrose du genou (gonarthrose) sont les suivants : Stade 0 radiographie normale ; Stade 1 ostéophyte de signification douteuse ; Stade 2 ostéophyte net sans modification de l'interligne articulaire ; Stade 3 ostéophyte net et diminution de l'interligne articulaire ; Stade 4 pincement sévère de l'interligne articulaire et sclérose de l'os sous-chondral.

Les stades radiologiques de l'arthrose de la hanche (coxarthrose) selon la classification de Kellgren et Laurence sont les suivants : Stade 0 : radiographie normale ; Stade 1 : pincement articulaire, ostéophytose péricapitale douteuse ; Stade 2 : pincement articulaire, ostéophytose, sclérose osseuse modérée ; Stade 3 : pincement articulaire net avec discrète ostéophytose, sclérose osseuse avec kyste, déformation de la tête fémorale et de l'acétabulum minime ; Stade 4 : disparition de l'espace articulaire avec sclérose osseuse et kyste, importante déformation de la tête fémorale et de l'acétabulum, avec ostéophytose majeure.

Le recours au scanner ou à l'IRM est intéressant et souvent réalisé en cas de symptomatologie clinique fortement évocatrice d'arthrose lorsque les radiographies sont normales. Ces examens d'imagerie sont plus sensibles et permettent de détecter des anomalies plus précoces non visibles sur les radiographies standards.

Dans la présente invention, l'arthrose peut toucher n'importe quelle articulation, notamment les articulations du rachis et les articulations périphériques, en particulier celles du genou (gonarthrose), de la hanche (coxarthrose), de la cheville, du pied, de la main, du poignet, du coude ou encore de l'épaule.

Selon un mode de réalisation, l'arthrose à traiter est une arthrose d'une articulation périphérique, de préférence une arthrose du genou.

Selon un mode de réalisation, l'arthrose à traiter est une arthrose primaire ou secondaire, de préférence est une arthrose primaire.

L'inhibiteur selon l'invention est de préférence utilisé dans le traitement de l'arthrose d'une articulation périphérique, en particulier d'une l'articulation du genou.

Selon un mode de réalisation, l'inhibiteur selon l'invention est utilisé dans le traitement d'une arthrose qui n'est pas une arthrose primaire.

### Sujet à traiter

Le sujet à traiter, ou patient, est un animal, de préférence un mammifère.

Selon un mode de réalisation, le sujet à traiter est un humain.

Le sujet humain est de préférence un adulte âgé de plus de 40 ans, de préférence de plus 50 ans.

Le sujet est de préférence un sujet présentant une arthrose de stade 2 ou 3 selon la classification de Kellgren et Lawrence.

Selon un autre mode de réalisation, le sujet est un animal domestique, de préférence un animal de compagnie, un animal destiné à une utilisation sportive, ou un animal de production.

L'animal de compagnie est de préférence choisi parmi le chat, ou le chien.

L'animal destiné à une utilisation sportive est de préférence le cheval, typiquement un cheval d'équitation.

L'animal de production est de préférence choisi parmi un bovin tel que la vache, le taureau ou le bœuf, un ovin tel que le mouton ou bien le porc.

Selon un mode de réalisation, le sujet à traiter ne présente pas de troubles du système nerveux central et périphérique, tels que la maladie de Parkinson, la schizophrénie et d'autres maladies liées à une carence en dopamine.

Selon un mode de réalisation, le sujet à traiter ne présente pas d'autres douleurs que celles liées à l'arthrose.

### Mode d'administration

L'inhibiteur utilisé selon l'invention peut être administré par toute voie d'administration connue, incluant notamment intra-articulaire, orale, ou sous-cutanée.

Selon un mode de réalisation particulièrement préféré, l'inhibiteur est administré par injection intra-articulaire, en particulier au niveau de l'articulation arthrosique.

Le terme « intra-articulaire », relatif au mode d'administration, désigne toute voie d'administration destinée à l'implantation ou l'injection d'un composé ou d'une composition à l'intérieur ou à proximité de l'espace intra-articulaire d'une articulation, en particulier d'une articulation arthrosique. De manière préféré, le terme « intra-articulaire » désigne un mode d'administration dans lequel l'inhibiteur est administré à l'intérieur de l'espace intra-articulaire d'une articulation arthrosique.

Les inventeurs ont montré que de façon particulièrement surprenante, l'administration intra-articulaire d'un inhibiteur de COMT permet de limiter la progression de la dégradation du cartilage.

De manière tout aussi surprenante, les inventeurs ont montré que l'administration intra-articulaire d'un inhibiteur de COMT permettait également de diminuer durablement la douleur pour le sujet, et ce même lorsque l'inhibiteur de COMT est administré de manière locale et non systémique.

Les inventeurs considèrent que l'administration intra-articulaire de l'inhibiteur selon l'invention présente l'avantage de maximiser l'efficacité de l'inhibiteur tant en ce qui concerne la limitation de la dégradation du cartilage que de la réduction de la douleur. Autrement dit, sans vouloir être liés par une théorie particulière, les inventeurs considèrent que l'administration intra-articulaire de l'inhibiteur selon l'invention présente l'avantage de permettre de diminuer la dose thérapeutiquement efficace relativement à d'autres voies d'administration telle que la voie orale. En outre, comme mentionné précédemment, les inventeurs considèrent que l'inhibiteur administré au niveau intra-articulaire permet une réduction de la douleur selon un mécanisme local et direct au niveau de l'articulation qui n'implique pas le système nerveux central ou périphérique.

Dans le cas d'une administration intra-articulaire, comme il sera détaillé ci-après, l'inhibiteur selon l'invention peut être administré en combinaison avec d'autres substances, en particulier des substances permettant la libération prolongée de l'inhibiteur, telles que l'acide hyaluronique.

L'inhibiteur utilisé selon l'invention peut également être administré par voie orale. L'administration orale est particulièrement préférée lorsque le sujet est un animal domestique, en particulier un chat ou un chien ou un cheval. Selon un mode de réalisation, le sujet est un animal domestique et l'administration est intra-articulaire.

L'inhibiteur utilisé selon l'invention est de préférence administré au sujet à une dose thérapeutiquement efficace. Le terme « dose thérapeutiquement efficace » tel qu'utilisé ici se réfère à la quantité nécessaire pour observer une activité thérapeutique ou préventive sur l'arthrose, en particulier la quantité nécessaire pour observer une inhibition ou un ralentissement de la destruction du cartilage arthrosique. La quantité d'inhibiteur à administrer ainsi que la durée du traitement peuvent être évaluées par l'homme du métier selon des critères tels que l'état physiologique, le sexe, l'âge, le poids du sujet à traiter, la nature de ou des articulations arthrosiques à traiter, l'inhibiteur choisi ainsi que la voie d'administration utilisée. La dose thérapeutiquement efficace pour chaque traitement peut être administrée sous la forme d'une dose unique ou de doses fractionnées.

L'inhibiteur utilisé selon l'invention peut être administré sous la forme d'une dose unique ou de doses fractionnées.

Selon un mode de réalisation, l'inhibiteur utilisé selon l'invention est de préférence un dérivé du nitrocatéchol notamment choisi dans le groupe constitué par la tolcapone, l'entacapone, l'opicapone et leurs mélanges, et administré au sujet par voie intra-articulaire.

Selon un mode de réalisation, l'inhibiteur utilisé selon l'invention est de préférence un dérivé du nitrocatéchol notamment choisi dans le groupe constitué par la tolcapone, l'entacapone, l'opicapone et leurs mélanges, et administré au sujet par voie orale.

L'inhibiteur utilisé selon l'invention peut être administré en combinaison avec d'autres substances actives. L'inhibiteur selon l'invention et la ou les autres substances actives peut être administrées simultanément (en même temps) ou séquentiellement (à des moments différents), par la même voie d'administration ou des voies d'administration différentes.

Dans le cade de la présente invention, on distingue notamment parmi les substances actives utiles dans le traitement de l'arthrose :
- les anti-arthrosiques symptomatiques d'effet rapide, qui ont une efficacité sur les symptômes douloureux de l'arthrose. On classe dans cette catégorie différents produits parmi lesquels les antalgiques tel que le paracétamol, les anti-inflammatoires non stéroïdiens tels que l'acéclofénac, l'acide niflumique, l'acide tiaprofénique, le célécoxib, le diclofénac, l'étodolac, l'étoricoxib, le flurbiprofène, l'ibuprofène, l'indométacine, le kétoprofène, le méloxicam, le nabumétone, le naproxène, le piroxicam, le sulindac ténoxicam, et les anti-inflammatoires stéroïdiens notamment de type corticoïdes, en particulier corticoïdes injectables à action immédiate ou prolongée tels que la bétaméthasone ;
- les anti-arthrosiques symptomatiques d'action lente, ou AASAL, qui ont une efficacité sur les symptômes douloureux de l'arthrose. On classe dans cette catégorie différents produits parmi lesquels le plasma riche en plaquettes (PRP), la chondroïtine par exemple la chondroïtine sulfate, la glucosamine par exemple la glucosamine phosphate ou sulfate, l'acide hyaluronique. La glucosamine et chondroïtine sont usuellement administrées par voie orale. L'acide hyaluronique et les PRP sont usuellement administrés par voie intra-articulaire ;
- les chondroprotecteurs ou anti-arthrosiques de fond, ou. Comme défini ci-dessus, les chondroprotecteurs ont une efficacité sur le ralentissement ou l'inhibition du processus de destruction dégradation du cartilage. Certains AASAL, tels que les PRP sont parfois considérés comme ayant une activité chondroprotectrice ; cela reste toutefois discuté.

Ainsi, selon un mode de réalisation, l'inhibiteur utilisé selon l'invention peut notamment être administré en combinaison avec une ou plusieurs substances actives choisie dans le groupe constitué des antalgiques tels que le paracétamol, des anti-inflammatoires non stéroïdiens tels que l'acéclofénac, l'acide niflumique, l'acide tiaprofénique, le célécoxib, le diclofénac, l'étodolac, l'étoricoxib, le flurbiprofène, l'ibuprofène, l'indométacine, le kétoprofène, le méloxicam, le nabumétone, le naproxène, le piroxicam, le sulindac ténoxicam ; des anti-inflammatoires stéroïdiens notamment de type corticoïdes, en particulier corticoïdes injectables à action immédiate ou prolongée tels que la bétaméthasone ; des antiarthrosiques symptomatiques d'action lente tels que le plasma riche en plaquettes (PRP), la chondroïtine par exemple la chondroïtine sulfate, et la glucosamine par exemple glucosamine phosphate ou sulfate ; des chondroprotecteurs.

### Deuxième objet de l'invention

L'inhibiteur de la présente description est de préférence administré sous la forme d'une composition pharmaceutique.

Ainsi, selon un second objet, l'invention concerne une composition pharmaceutique comprenant un ou plusieurs inhibiteurs de COMT selon le premier objet de l'invention, pour le traitement de l'arthrose et un excipient pharmaceutiquement acceptable.

On entend par « pharmaceutiquement acceptable » une substance qui n'est pas biologiquement ou autrement indésirable, c'est-à-dire qui peut être incorporée dans une composition pharmaceutique administrée à un patient ou un animal sans causer d'effets biologiques indésirables ou sans interagir de manière délétère avec l'un des autres composants de la composition dans laquelle elle est contenue, par exemple en inhibant ou diminuant les propriétés anti-inflammatoires de l'inhibiteur de COMT.

Typiquement, l'excipient pharmaceutiquement acceptable pourra être choisi parmi un diluant, un désintégrant, un liant, un agent de glissement, un agent lubrifiant, un agent mouillant, un agent tampon, un agent de suspension, un adjuvant, un émulsifiant, un absorbant, un conservateur, un agent de surface, un agent édulcorant, un antioxydant, ou un mélange de ceux-ci. Ces excipients sont par exemple décrits dans « The Science and Practice of Pharmacy 1995, edited by E. W. Martin, Mack Publishing Company, 19th edition, Easton, Pa ».

La quantité d'inhibiteur de COMT selon l'invention dans les compositions peut varier de manière à administrer une quantité efficace d'inhibiteur pour obtenir la réponse thérapeutique souhaitée pour un patient ou un animal domestique particulier.

La composition peut en outre comprendre une ou plusieurs autres substances actives telles que définies ci-dessus, de préférence choisies dans le groupe des corticoïdes injectables à action immédiate ou prolongée tels que la bétaméthasone ; des antiarthrosiques symptomatiques d'action lente tels que le plasma riche en plaquettes (PRP), la chondroïtine par exemple la chondroïtine sulfate, et la glucosamine par exemple glucosamine phosphate ou sulfate ; des chondroprotecteurs.

Le ou les inhibiteurs sont de préférence un ou plusieurs dérivés du nitrocatéchol notamment choisis dans le groupe constitué par la tolcapone, l'entacapone, l'opicapone et leurs mélanges.

La composition pharmaceutique utilisée selon l'invention est de préférence sous la forme d'une composition ingérable ou injectable, de préférence sous la forme d'une composition destinée à être administrée par voie intra-articulaire ou orale.

Dans un mode de réalisation, la composition pharmaceutique utilisée selon l'invention est une composition sous la forme d'une composition injectable destinée à être administrée par voie intra-articulaire.

Dans ce mode de réalisation, la composition se présente avantageusement sous la forme d'une composition à libération prolongée.

L'expression « à libération prolongée », relative à la formulation, désigne une formulation permettant de réduire la clairance de l'inhibiteur selon l'invention, ou autrement dit d'augmenter le temps de demi-vie de l'inhibiteur au niveau de l'articulation après son administration. Les expressions « à libération prolongée » ; « à libération contrôlée », « à libération différée », ou encore « à action prolongée » sont interchangeables dans la présente invention.

Une composition pharmaceutique à libération prolongée présente l'avantage d'augmenter l'efficacité thérapeutique de chaque dose, et dans le cadre d'une administration fractionnée, d'allonger l'intervalle de temps entre deux administrations.

Dans ce mode de réalisation, la composition pharmaceutique utilisée selon l'invention peut notamment comprendre l'inhibiteur selon l'invention et une fraction polymérique, l'inhibiteur étant de préférence dispersé dans la fraction polymérique.

La fraction polymérique peut notamment comprendre un polymère, de préférence un polymère biodégradable et/ou biocompatible, choisi dans le groupe incluant mais non limité à l'acide hyaluronique, le collagène, le chitosane, l'acide polylactique (PLA), l'acide glycolique, les copolymères d'acide lactique et d'acide glycolique (PLGA), les polyoxalates, la polycaprolactone, les polyesters, et leurs mélanges.

Dans un mode de réalisation, la fraction polymérique est sous la forme d'un gel ou apte à former un gel.

Le terme "gel" désigne un réseau polymère non fluide gonflé par un solvant.

L'expression « fraction polymérique apte à former un gel », se réfère à une fraction polymérique apte à gélifier *in situ,* c'est-à-dire à former un gel une fois placée dans l'environnement biologique dans lequel elle a été administrée, typiquement l'espace intra-articulaire.

La composition pharmaceutique de ce mode de réalisation peut par exemple se présenter sous la forme d'une solution comprenant le ou les inhibiteurs et la fraction polymérique ou bien d'une suspension de particules, typiquement de microparticules, dans laquelle les particules sont formées de la fraction polymérique sous forme d'un gel dans lequel sont encapsulés le ou les inhibiteurs.

Dans un mode particulier, le polymère, la fraction polymérique ou le gel est biocompatible et/ou biodégradable.

L'expression « biodégradable », relative au polymère, à la fraction polymérique ou au gel, signifie qu'in situ, c'est-à-dire dans l'articulation, le polymère, la fraction polymérique ou le gel est capable de se dégrader naturellement, dans des conditions physiologiques, permettant ainsi la libération de l'inhibiteur de manière prolongée. Les réactions impliquées lors de la biodégradation peuvent inclure des réactions d'hydrolyse, c'est-à-dire la rupture de liaisons covalentes par réaction avec l'eau. Ces réactions peuvent être catalysées par l'action d'enzymes naturellement présentes sur le site d'injection, typiquement au niveau de l'articulation.

Selon la présente divulgation, l'expression « biocompatible » relative au polymère, à la fraction polymérique ou au gel, signifie un polymère, une fraction polymérique ou un gel ayant la capacité de ne pas dégrader l'environnement biologique dans lequel elle est placée.

Selon un mode de réalisation, la composition pharmaceutique utilisée selon l'invention comprend un ou plusieurs inhibiteurs selon le premier objet de l'invention, de préférence un ou plusieurs dérivés du nitrocatéchol notamment choisis dans le groupe constitué par la tolcapone, l'entacapone, l'opicapone et leurs mélanges, et de l'acide hyaluronique.

### EXEMPLES

### Exemple 1 : Essais in vitro sur des cartilages humains

### Matériel et méthodes :

### Culture cellulaire :

Les cellules ont été obtenus à partir de déchets opératoires selon la réglementation en vigueur (protocole approuvé par le comité de protection des personnes (CPP) Nord-Ouest III). Après la signature du consentement libre et éclairé par les patients, des têtes fémorales, des membranes synoviales et de la moelle osseuse sont récupérées à l'issue d'opération d'arthroplastie de la hanche chez des patients arthrosiques.

Les têtes fémorales sont disséquées et des copeaux de cartilages sont réalisés. Les chondrocytes sont libérés par digestion à la pronase de type XIV (2 mg/ml pendant 15 minutes ; Sigma), suivie d'une digestion à la collagénase de type II (2 mg/ml pendant une nuit ; Gibco). Les cellules sont ensuite ensemencées à 4×10⁴ cellules/cm² dans du milieu Eagle modifié de Dulbecco (DMEM) supplémenté avec 10% de sérum de veau foetal (SVF) avec un cocktail d'antibiotiques. Elles sont ensuite incubées à 37°C dans une atmosphère humidifiée contenant 5% de CO₂.

Les synoviocytes sont obtenus à partir de membranes synoviales de patients subissant une chirurgie de remplacement de la hanche. Les synoviocytes sont libérées par digestion avec de la collagénase de type I (2 mg/ml pendant une nuit ; Gibco). Après rinçage au PBS, les cellules récupérées sont ensemencées avec du milieu DMEM complémenté avec 10% de SVF et un cocktail d'antibiotiques.

Les cellules de la moelle osseuse ont été fractionnées sur un gradient de densité Hypaque-Ficoll. Les cellules mononucléaires ont été isolées, ensemencées à la densité de 5.10⁴ cellules/cm² et cultivées en alpha-MEM additionné de 10% de sérum de veau fœtal (Invitrogen, Cergy-Pontoise, France), 2 mM L-glutamine, 1 ng/ml FGF-2 (Sigma Chemical Co) et des antibiotiques. A près de 80 % de confluence, les cellules ont été récoltées par trypsinisation (0,25 % trypsine/1 mm EDTA, Invitrogen) et ensemencées à 10³ cellules/cm². Après 5 passages, la disparition des marqueurs hématopoïétiques CD34 et CD45 a été vérifiée par RT-PCR et les cellules ont été utilisées pour les expériences.

Pour les trois types cellulaires, les cellules ont été amplifiées dans une enceinte humide à 37°C sous 5% de CO₂. Les milieux de culture sont changés 2 à 3 fois par semaine. Un test mycoplasme a été réalisé afin de vérifier qu'elles ne sont pas contaminées. Une partie a été traité avec de l'IL-1β 1ng/mL (Sigma) dilué dans du milieu DMEM avec 10% de SVF et 0,1% de pénicilline et streptomycine pendant 48h, en présence ou non de tolcapone (HY-17406, CliniSciences).

### Extraction ARNm et RT-PCR :

Les ARN totaux sont extraits à l'aide du kit RNeasy mini (Qiagen) selon les instructions du fabricant. 1 µg d'ARN subissent ensuite un traitement à la DNAse I (Sigma-Aldrich) puis sont rétro-transcrits en ADNc à l'aide de la transcriptase inverse du virus de leucémie murine de Moloney (Invitrogen) selon le protocole du fournisseur. Les ADNc sont dilués au 1/100ème et stockés à -20°C jusqu'à leur utilisation en PCR en temps réel.

Des amorces de PCR en temps réel utilisées en routine au laboratoire ou conçues à l'aide de l'outil Primer-BLAST (NCBI) vis-à-vis de RPL13A, IL6, MMP13 et COMT sont utilisées.

Les amorces sens et anti-sens sont diluées à 130 nM, avec 5 µl d'ADNc dilué au 1/100ème ainsi que 7.5 µl de Power SYBR Green master mix dans un volume réactionnel de 15 µL (Applied Biosystems). Les échantillons sont dénaturés à 95°C pendant 10 secondes, puis l'hybridation des amorces et la polymérisation ont lieu pendant 1 minute à 60°C et cela pendant 40 cycles. Ces cycles sont effectués en utilisant le système Step One Plus Real Time PCR (Applied Biosystems). Les niveaux d'expression relatifs des ARNm ont été calculés via la méthode du 2-ΔΔCT et sont normalisés à l'expression de RPL13A, un gène de ménage utilisé classiquement comme référence pour la normalisation de l'expression génique dans les cellules articulaires humaines. Afin de visualiser l'amplification, les échantillons après RT-PCR ont été déposé sur un gel d'agarose à 2%. Les signaux ont été révélés à l'aide d'un imageur Bio-Rad.

### Extraction protéique et Western-Blot :

Après traitement, les tapis cellulaires sont rincés au PBS, puis le tapis cellulaire est gratté dans un tampon de lyse dénommé « radio-immunoprécipitation » (RIPA : Tris-HCI 50 mM ; IGEPAL 1% ; NaCl 150 mM; EGTA 1 mM; NaF 1 mM) supplémenté avec des inhibiteurs de protéases (leupeptine, aprotinine, pepstatine, fluorure de phénylméthylsulfonyle (PMSF), et de phosphatases (orthovanadate de sodium Na₃VO₄), afin de lyser les cellules et extraire les protéines. Les lysats cellulaires obtenus sont incubés 30 minutes à 4°C, puis centrifugés 30 min à 10 000g à 4°C. Les surnageants contenant les protéines est récupérés et conservés à -20°C jusqu'à leur utilisation en western blot. La concentration protéique de chaque échantillon est ensuite déterminée selon la méthode de Bradford avec le réactif de Bradford Protein Assay (Biorad). Une gamme étalon est réalisée en duplicat et les échantillons en triplicat.

30 µg de protéines par échantillon migrent par électrophorèse sur un gel de dodécylsulfate de sodium - polyacrylamide (SDS-PAGE) et sont transférés sur des membranes de difluorure polyvinylidène (Bio-Rad). Après le blocage des sites non spécifiques, la membrane est incubée avec les anticorps primaires dirigés contre COMT (PA5-76864 ; Thermo-Fisher) dilués au 1/1000 pendant une nuit à 4°C. Après rinçage, les membranes ont été incubées avec des anticorps secondaires (SC- 516087, Santa-Cruz) couplés à la péroxydase pendant 1h à température ambiante. Après incubation avec le réactif oxydant et le luminol (1 minute sous agitation, Western Lightning ECL Pro, PerkinElmer. L'actine (sc-47778, Santa-Cruz) a été utilisée afin de vérifier le dépôt homogène des échantillons. Les signaux ont été révélés à l'aide d'un imageur (Bio-Rad).

### Protéomique :

Les expériences de protéomique ont été réalisé sous la forme d'une prestation de service sur la plateforme Proteogen de l'université de Caen Normandie. Les analyses ont été réalisées à partir d'échantillons protéiques issus du tapis cellulaire (extraction au RIPA) ou directement sur les milieux de culture, selon la procédure décrite dans l'article suivant (Brochard S, Pontin J, Bernay B, Boumediene K, Conrozier T, Baugé C. The benefit of combining curcumin, bromelain and harpagophytum to reduce inflammation in osteoarthritic synovial cells. BMC Complément Med Ther. 2021 Oct 14;21(1):261. doi: 10.1186/s12906-021-03435-7. PMID: 34649531; PMCID: PMC8515758.).

### Résultats :

L'expression de COMT dans différents types de cellules présentes au niveau de l'articulation (chondrocytes, synoviocytes, cellules souches mésenchymateuses) est analysée par RT-PCR et Western-blot (figure 1). Ces analyses révèlent que COMT est exprimé à la fois au niveau d'ARNm et de protéine dans les 3 types cellulaires étudiés. Ainsi, les résultats de RT-PCR montrent que les chondrocytes humains, les synoviocytes humains et les cellules souches mésenchymateuse issues de moelle osseuse humaine expriment l'ARNm de COMT. Par ailleurs, le Western-blot démontre une production des formes solubles (24kDa) et membranaires (30 kDa) de la protéine COMT par ces 3 types cellulaires.

Ayant démontré que COMT était exprimé dans les chondrocytes, nous avons étudié l'effet de l'inhibition de l'activité de COMT dans ces cellules stimulées à l'interleukine-1b (IL-1b). L'IL-1b est en effet classiquement utilisé pour modéliser l'arthrose dans des études in vitro. Cette cytokine pro-inflammatoire est présente dans l'articulation arthrosique et est impliquée dans la physiopathologie de l'arthrose (inflammation, catabolisme). Ainsi, des chondrocytes articulaires humains ont été traités avec des doses croissantes d'un inhibiteur pharmacologique de COMT, le tolcapone.

Nos expériences (figure 3) montrent clairement que cet inhibiteur de COMT s'oppose à l'action néfaste de l'IL-1b et réduit notamment l'expression de gènes impliqués dans le catabolisme (tel que MMP13) et de l'inflammation (tel que l'IL-6).

Afin d'avoir une vision plus globale de l'action des inhibiteurs de COMT sur le phénotype des chondrocytes, des expériences de protéomique ont été réalisé (). Les résultats de ces expériences (non représentés) montrent que l'inhibition pharmacologique de COMT affecte l'expression d'un grand nombre de protéines impliquées dans le processus arthrosique dans les chondrocytes articulaires humains. L'inhibition pharmacologique de COMT stimule notamment l'expression et la sécrétion de collagènes, notamment COL2.

Ces résultats confirment l'action anti-inflammatoire et anti-catabolique de l'inhibition de COMT dans des chondrocytes articulaires humains stimulés à l'IL-1 et montrent par ailleurs une action complémentaire sur l'anabolisme puisque nous identifions une augmentation de l'expression et de la sécrétion de la protéine collagène de type 2 (une protéine matricielle essentielle du cartilage).

Ces résultats montrent que cibler COMT pourrait avoir une action anti-catabolique, anti-inflammatoire et chondroprotectrice, et suggère une action directe sur les cellules présentes au niveau de l'articulation.

### Exemple 2 : Essais in vivo en modèle murin

### Matériel et méthodes :

### Animaux, induction de l'arthrose et traitements

Cette étude a été autorisée par le ministère de l'Enseignement Supérieur, de la Recherche et de l'Innovation après avis favorable du comité d'éthique locale, CENOMEXA (agrément n°B14118015). L'étude est réalisée au sein d'une animalerie agrée (UMS-CYCERON). Des souris C57BL7 mâles, âgées de 10 semaines ont été utilisés. Les souris étaient placées par cage de 8, en cycle inversé, nourriture et eau disponible *ad libidum.* L'état de santé de chaque souris était évalué quotidiennement.

Le modèle d'arthrose traumatique, reproduit par une déstabilisation du ménisque interne (DMM) de la souris est induit sous anesthésie générale. Après désinfection de la peau à l'alcool à 70°, la peau et le fascia sous cutané sont coupés aux ciseaux stériles. Ensuite, le ligament latéral interne a été coupé transversalement et les fibres du muscle vaste interne sont sectionnées dans le sens longitudinal pour permettre la luxation externe de la patella. Puis, la méniscectomie du ménisque interne de genou droit est réalisée sous loupe. Enfin, le muscle vaste interne et la peau ont été suturé. Un groupe contrôle, dit « Sham » est constitué par des souris subissant la même intervention sans méniscectomie, a été établi.

Les injections intra-articulaires (MIA ou tolcapone) ont également été réalisées sous anesthésie générale. Après un repérage clinique du ligament patellaire, les injections sont faites par voie intra articulaire du genou après passage du ligament patellaire à l'aiguille 27 Gauge.

### Immunohistologie

Des genoux de souris ont été fixés dans du PFA à 4 % pendant 24 h et décalcifiés dans de l'EDTA 0,5 M pH 8,0 pendant 10 jours avant d'être inclus dans de la paraffine. Des coupes sagittales de 5µm d'épaisseur ont été réalisées et monté sur des lames de microscopies SuperFrost^{®} Silanisées. Avant utilisation les lames sont déparafinées par 3 bains de 15 min de xylène. Puis réhydratées dans des bains successifs de 100%, 70% et 40% d'éthanol puis deux bains successifs d'eau et de PBS. Un démasquage antigénique est réalisé par chaleur (70°) dans un tampon citrate (pH 6) durant 4h, suivi d'une digestion à la hyaluronidase (1mg/ml dans du PBS). Après 2 rinçages au PBS les peroxydases endogènes sont inhibées par une incubation de H₂O₂ à 3% à température ambiante durant 10 minutes. Les sites non spécifiques sont bloqués par une incubation à température ambiante de 30 minutes d'une solution de blocages (2,5% de sérum de cheval, 2,5% de BSA dans du PBS). L'anticorps, polyclonal de lapin dirigé contre COMT (PA5-76864 ; Thermo-Fisher) est utilisé au 1/500 dilué dans de la solution de blocage dilué au demi, son incubation se fait sur la nuit à 4°. La détection se fait grâce au kit ImmPRESS staining reagent (Vector Laboratories, France) avec un anticorps anti lapin. La révélation est réalisé en utilisant le kit diaminobenzidine substrate kit for peroxydase (Vector Laboratories, France). Pour finir une contre coloration à l'eau bleuté (trois goutes de bleu de toluidine a 1% dans 200ml d'eau) avant montage à l'ENTELAN.

### Histologie

Les articulations sont placées dans une solution de fixation (formaldehyde 37%, PBS) pendant 3 jours, puis décalcifié à l'aide d'Osteosoft pendant 48h. Ensuite, les articulations sont conservées à -80°C dans l'Optimal Cutting Temperature compound (OCT). Des coupes de 10 µm d'épaisseur ont été réalisées à -25°C à l'aide d'un Cryostat (CM 3050 S, Leica BIOSYSTEMS, France), puis montées sur des lames de microscopies SuperFrost^{®}. Les lames sont ensuite colorées au Fast Green puis à la safranine-O. La safranine-O permet de mettre en évidence les composants du cartilage articulaire. Les glycosaminoglycanes et protéoglycanes apparaissent en rouge. Le Fast Green sert de contre-colorant et colore en vert l'os minéralisé.

### Von Frey :

L'allodynie (douleur déclenchée pour un stimulus qui est normalement indolore) mécanique est évaluée par le test des filaments de Von Frey (BIOSEB In Vivo Research Instruments^{®}). Ce test repose sur le principe selon lequel lorsque l'extrémité du filament (de longueur et de diamètre donnés) est appliquée perpendiculairement, la force exercée par le filament augmente jusqu'à ce que le filament fléchisse. Au-delà, la force exercée par le filament reste constante. Pour ce test, les souris sont placées dans une cage individuelle, sur une grille. Une période d'habituation de 30 minutes était requise avant le début du test. Des filaments exerçant une force de plus en plus grande sont appliqués sous la patte de la souris, du bas vers le haut, perpendiculairement à la surface plantaire de la souris jusqu'à ce que le filament commence à fléchir. Le retrait de la patte, un sursaut ou le léchage de la patte sont interprétés comme des réponses positives, synonyme de douleur ou de gêne.

Les résultats sont présentés comme la valeur obtenue sur la patte arthrosique rapportée à la sensibilité de la patte controlatérale (non arthrosique).

### Résultats :

Nous avons poursuivi notre étude en étudiant l'expression protéique de COMT au niveau de l'articulation de souris normale et arthrosique (figure 2). Les résultats d'immunohistochimie montrent que l'expression de COMT est augmentée dans l'articulation arthrosique comparée à une articulation saine et que COMT est principalement exprimé par les chondrocytes.

L'effet du tolcapone a ensuite été testé *in vivo.* Pour cela, des injections intra-articulaires de tolcapone ont été réalisées à 1, 2, 3 et 4 semaines après l'induction de l'arthrose dans les souris. La douleur a été évaluée tout au long de l'expérience par analyse de Von Frey. L'étude histologique a été faite à 8 semaines (voir le plan expérimental, figure 4A).

Nos résultats montrent clairement un effet bénéfique de la tolcapone sur la progression de l'arthrose. D'une part, nous observons que l'injection intra-articulaire de tolcapone au niveau de l'articulation arthrosique, réduit nettement la douleur induite (figure 4B), tout en améliorant la structure de l'articulation (figure 5). Ainsi, l'étude histologique montre clairement que les injections intra-articulaires de l'inhibiteur de COMT au niveau de l'articulation arthrosique a réduit les dommages articulaires et très fortement limité la dégradation du cartilage dans ces souris (« MIA tolcapone ») comparés aux souris arthrosiques non traitées (« MIA+vehicule »).

Ces résultats démontrent que l'inhibition de COMT au niveau de l'articulation est utile dans le traitement de l'arthrose. Non seulement, elle ralentit la progression de l'arthrose (effet chondroprotecteur) mais elle réduit également simultanément la douleur induite.

## Revendications

1. Inhibiteur de la catéchol O-méthyltransférase (COMT) pour une utilisation dans le traitement de l'arthrose.

2. Inhibiteur pour une utilisation selon la revendication 1, dans lequel l'inhibiteur est destiné à ralentir la progression de l'arthrose et à réduire la douleur induite par l'arthrose.

3. Inhibiteur pour une utilisation selon l'une quelconque de la revendication 1 ou 2, dans lequel l'inhibiteur est choisi parmi un composé dérivé du nitrocatéchol, un anticorps ou un ARN interférent.

4. Inhibiteur pour une utilisation selon l'une quelconque des revendications 1 à 3 dans lequel l'inhibiteur est un dérivé du nitrocatéchol comprenant une partie catéchol portant un groupe nitro en position ortho ou para par rapport à l'un des groupes hydroxy de la partie catéchol.

5. Inhibiteur pour une utilisation selon l'une quelconque des revendications 3 ou 4, dans lequel le dérivé du nitrocatéchol est choisi dans le groupe constitué par la tolcapone, l'entacapone, l'opicapone, la nitecapone, la nebicapone, et leurs mélanges.

6. Inhibiteur pour une utilisation selon l'une quelconque des revendications 1 à 5, dans lequel l'inhibiteur est destiné à être administré par voie intra-articulaire, orale, ou sous-cutanée.

7. Inhibiteur pour une utilisation selon l'une quelconque des revendications 1 à 6, dans lequel l'inhibiteur est destiné à être administré par voie intra-articulaire.

8. Inhibiteur pour une utilisation selon l'une quelconque des revendications 1 à 7, dans lequel l'inhibiteur administré en combinaison avec une ou plusieurs substances actives choisies dans le groupe constitué des antalgiques, des anti-inflammatoires non stéroïdiens, des anti-inflammatoires stéroïdiens, et des anti-arthrosiques, typiquement des anti-arthrosiques symptomatiques d'action lente ou des chondroprotecteurs.

9. Composition pharmaceutique comprenant un ou plusieurs inhibiteurs de COMT tels que définis dans l'une quelconque des revendications 1 à 7, et un excipient pharmaceutiquement acceptable, pour une utilisation dans le traitement de l'arthrose.

10. Composition pharmaceutique pour une utilisation selon la revendication 9, dans lequel la composition est destiné à être administrée par voie intra-articulaire, orale, ou sous-cutanée.

11. Composition pharmaceutique pour une utilisation selon l'une quelconque des revendications 9 ou 10, comprenant en outre une ou plusieurs autres substances actives choisies dans le groupe constitué des antalgiques, des anti-inflammatoires non stéroïdiens, des anti-inflammatoires stéroïdiens et des anti-arthrosiques, typiquement des anti-arthrosiques symptomatiques d'action lente ou des chondroprotecteurs.

12. Composition pharmaceutique pour une utilisation selon l'une quelconque des revendications 9 à 11, destinée à être administrée par voie intra-articulaire.

13. Composition pharmaceutique pour une utilisation selon la revendication 12, comprenant l'inhibiteur et des anti-arthrosiques tels que le plasma riche en plaquettes (PRP), la chondroïtine, et la glucosamine.

14. Composition pharmaceutique pour une utilisation selon l'une quelconque des revendications 9 à 13, formulée sous la forme d'une composition à libération prolongée.

15. Composition pharmaceutique pour une utilisation selon l'une quelconque des revendications 9 à 13, comprenant l'inhibiteur dispersé dans une fraction polymérique comprenant un polymère biodégradable et/ou biocompatible de préférence choisi dans le groupe constitué par l'acide hyaluronique, le collagène, le chitosane, l'acide polylactique (PLA), l'acide glycolique, les copolymères d'acide lactique et d'acide glycolique (PLGA), les polyoxalates, la polycaprolactone, les polyesters, et leurs mélanges.
